(19) Europäisches Patentamt
European Patent Office
Office européen des brevets

(11) Veröffentlichungsnummer : **0 592 366 A1**

# (12) EUROPÄISCHE PATENTANMELDUNG

(21) Anmeldenummer : **93810689.5**

(22) Anmeldetag : **29.09.93**

(51) Int. Cl.[5] : **C07D 333/74,** C07D 209/60, C07D 209/88, C07D 307/92, C09K 9/02, C09B 5/26, C09B 5/24, G03C 1/73

(30) Priorität : **08.10.92 CH 3141/92**

(43) Veröffentlichungstag der Anmeldung : **13.04.94 Patentblatt 94/15**

(84) Benannte Vertragsstaaten : **BE CH DE FR GB IT LI NL SE**

(71) Anmelder : **CIBA-GEIGY AG**
**Klybeckstrasse 141**
**CH-4002 Basel (CH)**

(72) Erfinder : **Fischer-Reimann, Evelyn, Dr.**
**Schutzackerstrasse 64**
**D-79576 Weil am Rhein (DE)**

## (54) Photochrome Verbindungen, deren Herstellung und deren Verwendung.

(57) Verbindungen der Formeln I und II oder Gemische von Verbindungen der Formel I und Verbindungen der Formel II sowie deren Anachinone,

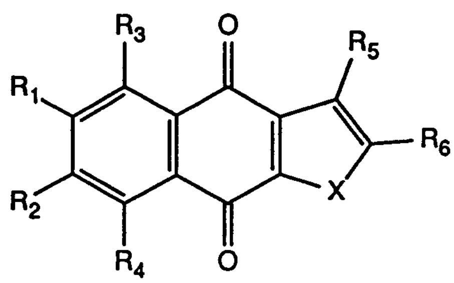

(I),

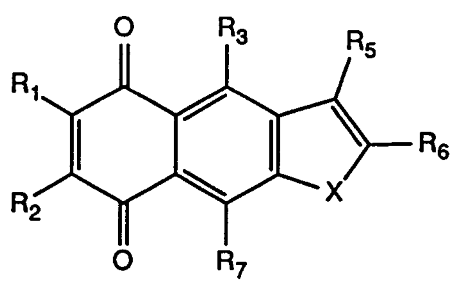

(II),

worin
X für -O-, -S- oder $-NR_8-$ steht,
$R_1$ und $R_2$ unabhängig voneinander H, $C_1$-$C_4$-Alkyl, $C_1$-$C_4$-Alkoxy, Phenyl, Benzyl, $-OR_9$, -CN, $-CO_2R_{10}$, $-CO-R_{11}$, Halogen, oder $R_1$ und $R_2$ zusammen $-CH=CR_{12}-CR_{13}=CH-$ bedeuten ;
$R_3$ die Gruppe $-OR_9$ und $R_4$ H, Halogen oder die Gruppe $-OR_9$ darstellen ;
$R_5$ und $R_6$ unabhängig voneinander die gleiche Bedeutung wie $R_1$ und $R_2$ haben oder $R_5$ und $R_6$ zusammen 1,2-Benz-buta-1,3-dien-1,4-diyl bedeuten ;
$R_7$ H, Halogen oder die Gruppe $-OR_9$ darstellt ;
$R_8$ H, lineares oder verzweigtes $C_1$-$C_{18}$-Alkyl oder unsubstituiertes oder mit $C_1$-$C_4$-Alkyl oder $C_1$-$C_4$-Alkoxy substituiertes Phenyl oder Benzyl bedeutet ;
$R_9$ unsubstituiertes oder mit $C_1$-$C_{12}$-Alkyl, $C_1$-$C_{12}$-Alkoxy, $C_1$-$C_{12}$-Alkylthio, $C_1$-$C_{12}$-Alkylsulfinyl, $C_1$-$C_{12}$-Alkylsulfonyl, Phenyl, Benzyl, -CN, $-CF_3$, Halogen, $-CO-R_{11}$ oder $-CO_2R_{10}$ substituiertes $C_6$-$C_{14}$-Aryl darstellt ;
$R_{10}$ für H, $C_1$-$C_{18}$-Alkyl,Cyclohexyl, Cyclopentyl, Phenyl, $C_1$-$C_{12}$-Alkylphenyl, Benzyl oder $C_1$-$C_{12}$-Alkylbenzyl steht ;
$R_{11}$ H, $C_1$-$C_4$-Alkyl oder Phenyl bedeutet ; und

EP 0 592 366 A1

$R_{12}$ und $R_{13}$ unabhängig die Bedeutung von $R_1$ und $R_2$ haben, sind photochrom und eignen sich zur Kontrastbildung, Lichtabsorption und zur Aufzeichnung von Informationen.

Die vorliegende Erfindung betrifft mindestens tricyclische heteroaromatische Paradione mit einem Furan-, Thiophen- oder Pyrrolring, ihre entsprechenden Anachinone, ein Verfahren zu deren Herstellung und deren Verwendung als photochrome Systeme zur Kontrastbildung, zur Lichtabsorption und zur Aufzeichnung von Informationen.

In der EP-A-0 438 376 sind photochrome 5,12-Naphthacendione beschrieben, deren Photochromismus - ein Farbumschlag von gelb nach rot - durch zwei Aryloxygruppen je in den 6,11-Stellungen bedingt ist.

In der EP-A-0 489 689 sind ähnliche photochrome 5,12-Naphthacendione beschrieben, die zusätzlich in den 2-, 3-, 8- und/oder 9-Stellungen mindestens eine weitere Aryloxygruppe oder Halogenatome enthalten.

In der EP-A-0 494 689 sind photochrome Benzothioxanthonoxide beschrieben, die in 11-Stellung und gegebenenfalls in 6-Stellung mit einer Aryloxygruppe substituiert sind.

Es wurde nun überraschend gefunden, dass auch mindestens tricyclische aromatische Verbindungen photochrom sind und einen deutlichen Farbumschlag von farblos bis gelb nach rot, violett oder blau aufweisen, wenn sie in Nachbarschaft zu einem Chinonring eine Furan-, Thiophen- oder Pyrrolgruppe ankondensiert ist und in mindestens einer Peristellung eines an den Chinonring ankondensierten Benzolrings eine Aryloxygruppe gebunden ist, oder wenn die kondensierten Ringe die Folge Chinonring/Peri-diaryloxybenzolring/Furan-, Thiophen- oder Pyrrolring aufweisen.

Ein Gegenstand der Erfindung sind Verbindungen der Formeln I und II oder Gemische von Verbindungen der Formel I und Verbindungen der Formel II,

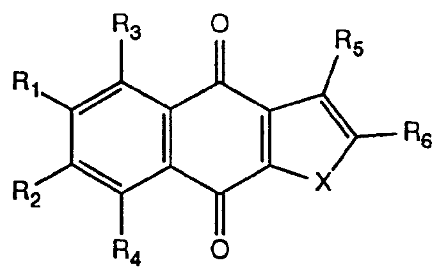

(I),

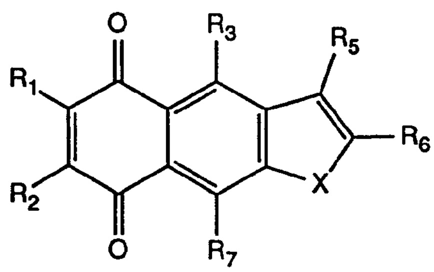

(II),

worin

X für -O-, -S- oder $-NR_8-$ steht,

$R_1$ und $R_2$ unabhängig voneinander H, $C_1$-$C_4$-Alkyl, $C_1$-$C_4$-Alkoxy, Phenyl, Benzyl, $-OR_9$, -CN, $-CO_2R_{10}$, $-CO-R_{11}$, Halogen, oder $R_1$ und $R_2$ zusammen $-CH=CR_{12}-CR_{13}=CH-$ bedeuten;

$R_3$ die Gruppe $-OR_9$ und $R_4$ H, Halogen oder die Gruppe $-OR_9$ darstellen;

$R_5$ und $R_5$ unabhängig voneinander die gleiche Bedeutung wie $R_1$ und $R_2$ haben oder $R_5$ und $R_6$ zusammen 1,2-Benz-buta-1,3-dien-1,4-diyl bedeuten;

$R_7$ H, Halogen oder die Gruppe $-OR_9$ darstellt;

$R_5$ H, lineares oder verzweigtes $C_1$-$C_{18}$-Alkyl oder unsubstituiertes oder mit $C_1$-$C_4$-Alkyl oder $C_1$-$C_4$-Alkoxy substituiertes Phenyl oder Benzyl bedeutet;

$R_9$ unsubstituiertes oder mit $C_1$-$C_{12}$-Alkyl, $C_1$-$C_{12}$-Alkoxy, $C_1$-$C_{12}$-Alkylthio, $C_1$-$C_{12}$-Al-kylsulfinyl, $C_1$-$C_{12}$-Alkylsulfonyl, Phenyl, Benzyl, -CN, $-CF_3$, Halogen, $-CO-R_{11}$ oder $-CO_2R_{10}$ substituiertes $C_6$-$C_{14}$-Aryl darstellt;

$R_{10}$ für H, $C_1$-$C_{18}$-Alkyl, Cyclohexyl, Cyclopentyl, Phenyl, $C_1$-$C_{12}$-Alkylphenyl, Benzyl oder $C_1$-$C_{12}$-Alkylbenzyl steht;

$R_{11}$ H, $C_1$-$C_4$-Alkyl oder Phenyl bedeutet; und

$R_{12}$ und $R_{13}$ unabhängig die Bedeutung von $R_1$ und $R_2$ haben.

Bei $R_1$ und $R_2$ in der Bedeutung von Alkyl und Alkoxy kann es sich um Methyl, Ethyl, n-oder i-Propyl, n-, i- oder t-Butyl, Methoxy, Ethoxy, n- oder i-Propoxy oder n-, i- oder t-Butoxy handeln. Bevorzugt sind Methyl,

Ethyl, Methoxy und Ethoxy.

Bei $R_1$ und $R_2$ in der Bedeutung von Halogen handelt es sich bevorzugt um F, Cl oder Br, und besonders bevorzugt um Cl.

In einer bevorzugten Ausführungsform der Erfindung bedeuten $R_1$ und $R_2$ H oder die Gruppe $R_9$, oder $R_1$ und $R_2$ zusammen die Gruppe -CH=CH-CH=CH-.

$R_4$ und $R_7$ in der Bedeutung von Halogen stellen bevorzugt F, Cl oder Br und insbesondere bevorzugt Cl dar.

In einer anderen bevorzugten Ausführungsform der Erfindung bedeuten $R_5$ und $R_5$ H, $C_1$-oder $C_2$-Alkyl, $C_1$- oder $C_2$-Alkoxy, -CN, -$CO_2$-($C_1$-$C_4$-Alkyl), -CO-($C_1$-$C_4$-Alkyl), Phenyl, Benzyl, oder $R_5$ und $R_6$ zusammen 1,2-Benz-buta-1,3-dien-1,4-diyl oder die Gruppe -CH=$CR_{12}$-$CR_{13}$=CH-, worin $R_{12}$ und $R_{13}$ für H, $C_1$- oder $C_2$-Alkyl, $C_1$- oder $C_2$-Alkoxy oder -$CO_2$-($C_1$-$C_4$-Alkyl) stehen. Eines von $R_{12}$ und $R_{13}$ stehen besonders bevorzugt für H.

Bei $R_5$ in der Bedeutung von Alkyl kann es sich um $C_1$-$C_{12}$-Alkyl, bevorzugt $C_1$-$C_4$-Alkyl handeln. Beispiele sind Methyl, Ethyl und die Isomeren von Propyl, Butyl, Pentyl, Hexyl, Heptyl, Octyl, Nonyl, Decyl, Undecyl, Dodecyl, Tetradecyl, Hexadecyl und Octadecyl. Bevorzugt ist $R_8$ als Alkyl Methyl oder Ethyl. Einige Beispiele für $R_8$ als substituiertes Phenyl oder Benzyl sind Methylphenyl, Ethylphenyl, Methylbenzyl, Ethylbenzyl, Methoxyphenyl, Ethoxyphenyl, Methoxybenzyl und Ethoxybenzyl.

In einer bevorzugten Untergruppe stellt $R_5$ $C_1$-$C_4$-Alkyl, Phenyl oder Benzyl dar.

$R_9$ in der Gruppe -$OR_9$ ist bevorzugt unsubstituiertes oder substituiertes $C_6$-$C_{10}$-Aryl, zum Beispiel Phenyl, 1- oder 2-Naphthyl. Bevorzugt stellt $R_9$ unsubstituiertes oder substituiertes Phenyl dar.

Die Gruppe $R_9$ kann mit einem oder mehreren, bevorzugt 1 bis 3 Resten substituiert sein. Wenn $R_9$ mit Alkyl, Alkoxy, Alkylthio, Alkylsulfinyl oder Alkylsulfonyl substituiert ist, kann das Alkyl linear oder verzweigt sein und bevorzugt 1 bis 6, besonders 1 bis 4 C-Atome enthalten. Beispiele sind Methyl, Ethyl, die Isomeren von Propyl, Butyl, Pentyl, Hexyl, Heptyl, Octyl, Nonyl, Decyl, Undecyl, Dodecyl und die entsprechenden Alkoxy-, Alkylthio-, Alkylsulfinyl- und Alkylsulfonylreste. Bevorzugt sind Methyl, Ethyl, n- und i-Propyl, n-, i- und t-Butyl, Methoxy, Ethoxy, Methylthio, Ethylthio, Methyl- und Ethylsulfinyl, sowie Methyl- und Ethylsulfonyl.

Wenn $R_9$ mit Halogen substituiert ist, handelt es sich bevorzugt um -Br, -Cl und -F.

In einer besonders bevorzugten Ausführungsform stellt $R_9$ Phenyl dar.

$R_{10}$ in der Bedeutung von Alkyl kann linear oder verzweigt sein. Beispiele für Alkyl sind zuvor genannt worden. Bevorzugt enthält $R_{10}$ als Alkyl 1 bis 12, besonders 1 bis 6 C-Atome. $R_{10}$ als Alkylphenyl ist bevorzugt $C_1$-$C_6$-, besonders $C_1$-$C_4$-Alkylphenyl, z. B. Dodecylphenyl, Octylphenyl, Hexylphenyl, n-, i- oder t-Butylphenyl, n- oder i-Propylphenyl, Ethylphenyl, Methylphenyl. $R_{10}$ als Alkylbenzyl ist bevorzugt $C_1$-$C_6$-, besonders $C_1$-$C_4$-Alkylbenzyl, z.B. Dodecylbenzyl, Octylbenzyl, Hexylbenzyl, n-, i- oder t-Butylphenyl, n-oder i-Propylbenzyl, Ethylbenzyl, Methylbenzyl. $R_{10}$ ist bevorzugt H oder $C_1$-$C_{18}$-Alkyl, besonders $C_1$-$C_{12}$-Alkyl und ganz besonders $C_1$-$C_4$-Alkyl.

$R_{11}$ stellt bevorzugt H, Methyl, Ethyl oder Phenyl dar.

$R_{12}$ und $R_{13}$ stehen bevorzugt für H, $C_1$- oder $C_2$-Alkyl, $C_1$- oder $C_2$-Alkoxy oder -$CO_2$-($C_1$-$C_4$-Alkyl). Besonders bevorzugt steht eines von $R_{12}$ und $R_{13}$ für H und das andere von $R_{12}$ und $R_{13}$ für $C_1$- oder $C_2$-Alkyl, $C_1$- oder $C_2$-Alkoxy oder -$CO_2$-($C_1$-$C_4$-Alkyl). Besonders bevorzugte Reste für $R_{12}$ und/oder $R_{13}$ sind Methoxy, Ethoxy, -C(O)$OCH_3$ und -C(O)$OC_2H_5$.

Besonders bevorzugt sind Verbindungen der Formeln I und II, worin $R_1$ und $R_2$ H oder -$OC_6H_5$ oder $R_1$ und $R_2$ zusammen -CH=CH-CH=CH- bedeuten, $R_3$ -$OC_6H_5$ und $R_4$ und $R_7$ H, Cl oder -$OC_6H_5$ darstellen, $R_5$ und $R_6$ unabhängig voneinander H, -CN, Methyl, Ethyl, -$C_6H_5$ oder -CO-$CH_3$ oder -C(O)-$C_2H_5$ oder $R_5$ und $R_5$ zusammen 1,2-Benz-buta-1,3-dien-1,4-diyl oder die Gruppe -CH=$CR_{12}$-$CR_{13}$=CH- bedeuten, worin $R_{12}$ und $R_{13}$ unabhängig voneinader H, Methyl, Ethyl, Methoxy, Ethoxy, -$CO_2CH_3$ oder -$CO_2C_2H_5$ sind, und X -O-, -S- oder -$NR_5$- darstellt, worin $R_5$ $C_1$-$C_4$- Alkyl, Benzyl oder Phenyl ist.

Ein weiterer Gegenstand der Erfindung ist ein Verfahren zur Herstellung von Verbindungen der Formel I und II, das dadurch gekennzeichnet ist, dass man eine Verbindung der Formeln III oder IV,

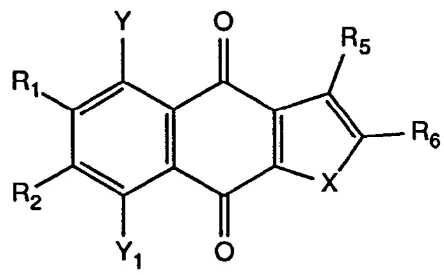

(III),

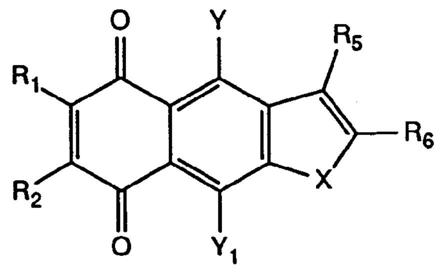

(IV),

worin $R_1$, $R_2$, $R_5$, $R_5$ und X die zuvor angegebenen Bedeutungen haben, Y für Halogen oder $-NO_2$ steht und $Y_1$ H, Halogen oder $-NO_2$ bedeutet, in Gegenwart eines polaren aprotischen oder protischen Lösungsmittels und bei erhöhter Temperatur mit einer Verbindung der Formel $R_9O^{\ominus}M^{\oplus}$ umsetzt, worin $R_9$ die zuvor angegebenen Bedeutungen hat und $M^{\oplus}$ für ein Alkalimetall- oder ein Ammoniumkation steht.

Y und $Y_1$ stellen als Halogen bevorzugt F, Cl oder Br und besonders bevorzugt Cl dar.

Beispiele für $M^{\oplus}$ sind $Li^{\oplus}$, $Na^{\oplus}$, $K^{\oplus}$ und tertiäres Ammonium, zum Beispiel Triethyl-, Trimethyl-, Tri-n-propyl- oder Tri-n-butylammonium.

Das erfindungsgemässe Verfahren wird bevorzugt bei Temperaturen von 50 bis 200°C, besonders 50 bis 150°C durchgeführt. Die Salze der Formel $R_9O^{\ominus}M^{\oplus}$ können als solche eingesetzt werden oder durch Umsetzung eines entsprechenden Phenols mit einer Alkalimetallbase oder Alkalimetallcarbonaten oder Aminen in situ im Reaktionsgemisch erzeugt werden. Die Salze können in äquimolaren Mengen oder im Ueberschuss, z.B. bis zu 40 Mol-% Ueberschuss eingesetzt werden.

Geeignete Lösungsmittel sind z.B. N-substituierte Carbonsäureamide und Lactame (z.B. Dimethylformamid oder N-Methylpyrrolidon), Sulfoxide und Sulfone (z.B. Dimethylsulfoxid, Tetramethylensulfon) oder Ether (z.B. n-Dipropylether, n-Dibutylether, Tetrahydrofuran oder Dioxan). Die Reaktion kann auch in einem Überschuss eines Phenols $R_9OH$ durchgeführt werden, das dann gleichzeitig als Lösungsmittel dient.

Die Isolierung und Reinigung der Verbindungen der Formeln I und II erfolgt nach üblichen Methoden, z.B. durch Kristallisation und Umkristallisation, oder chromatographische Verfahren.

Ein anderer Gegenstand der Erfindung sind die Verbindungen der Formeln III und IV

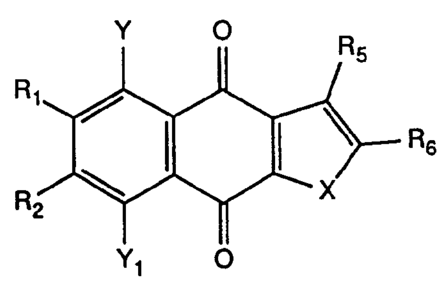

(III),

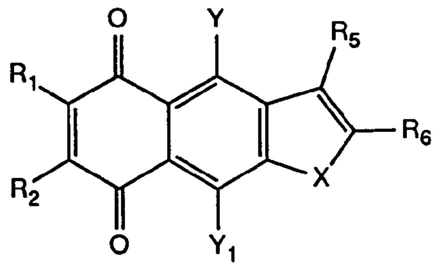

(IV),

worin $R_1$, $R_2$, $R_5$, $R_5$ und X die zuvor angegebenen Bedeutungen haben, Y für Halogen oder $-NO_2$ steht und $Y_1$ H, Halogen oder $-NO_2$ bedeutet.

Die Verbindungen der Formeln III und IV, worin Y und gegebenenfalls $Y_1$ Halogen bedeuten, sind zum Beispiel in bekannter Weise durch die Halogenierung mit geeigneten Halogenierungsmitteln wie zum Beispiel $POCl_3$ oder $POBr_3$ der miteinander im Gleichgewicht stehenden Hydrochinone der Formeln A und B erhältlich:

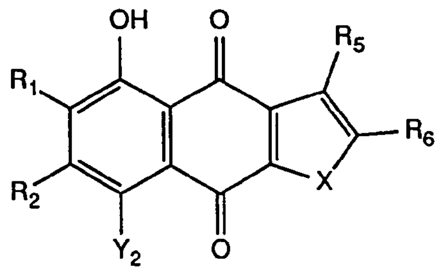

(A),

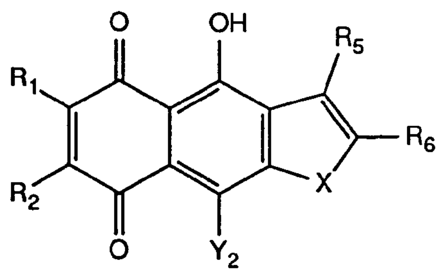

(B).

$Y_2$ bedeutet H oder -OH. Bei der Herstellung können Gemische dieser Isomeren entstehen und weiterverwendet werden; es ist aber auch möglich, die einzelnen Isomeren in reiner Form herzustellen, in dem man das Gleichgewicht zu einem Isomeren durch Behandlung mit Basen beziehungsweise Säuren beeinflusst.

Die Verbindungen der Formeln A und B können in an sich bekannter Weise [siehe zum Beispiel Ch. Weizmann et al., J. Chem. Soc., Seite 398 (1939)] durch Friedel-Crafts-Reaktionen von heterocyclischen 1,2-Dicarbonsäuren der Formel C

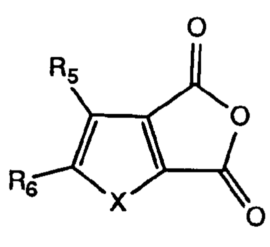

(C)

mit Mono- oder Diphenolen der Formel D

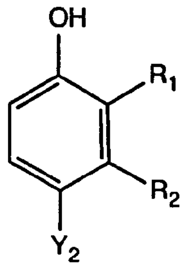

(D)

erhalten werden.

Die Verbindungen der Formeln III und IV können auch durch intramolekulare Friedel-Crafts-Reaktionen aus aromatischen Dicarbonsäureanhydriden der Formel E

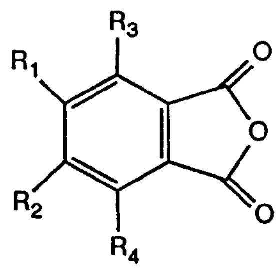

(E)

mit fünfgliedrigen Heteroaromaten der Formel F

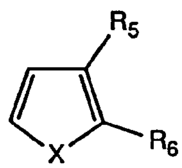

(F)

hergestellt werden, siehe zum Beispiel H.E. Schröder et al., JACS 74, Seite 4357 (1952) und A. T. Peters et al., J. Chem. Soc., Seite 1525 (1957).

Bei der Herstellung können Gemische von Stellungsisomeren gebildet werden, die man als solche zur Weiterreaktion verwenden kann oder die man mittels an sich bekannter Verfahren wie zum Beispiel fraktionierte Kristallisation oder chromatographische Verfahren vor der Weiterreaktion trennen kann.

Weitere Herstellverfahren sind in den Beispielen beschrieben.

Die Verbindungen der Formeln I und II sind kristallin, thermisch stabil und farblos bis gelb gefärbt. Sie sind in organischen Lösungsmitteln und in Polymeren löslich. Sie sind wirksame Farbindikatoren für photopolymerisierbare Systeme, die ethylenisch ungesättigte Doppelbindungen enthalten. Ferner sind die Verbindungen der Formel I reversibel photochrom.

Bei Bestrahlung der erfindungsgemässen Verbindungen, gegebenenfalls in einem Substrat, mit Licht einer Wellenlänge von etwa 300 bis 450 nm wird eine ausgeprägte Farbänderung nach rot, violett oder blau beobachtet. Die Lichtabsorption ist im Vergleich zu höherer Wellenlänge verschoben. Die Farbänderung beruht auf der photochemischen Umwandlung der erfindungsgemässen Parachinone in die entsprechenden Anachinone der Formeln V und VI. Die Umwandlungsgeschwindigkeit ist überraschend hoch und kann je nach Menge, Dicke der Probe und Strahlungsintensität unter 3 Sekunden liegen. Die Anachinone der Formeln V und VI sind somit durch Bestrahlung der Parachinone der Formeln I und II erhältlich. Die photochemische Umwandlung ist reversibel; werden die Anachinone im Wellenlängenbereich der neuen längerwelligen Absorptionbande bestrahlt, so wird eine Rückreaktion zu den farblos bis gelb gefärbten Parachinonen beobachtet.

Ein weiterer Gegenstand der Erfindung sind die Anachinone der Formeln V und VI

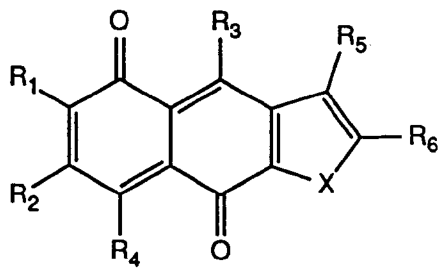

(V),

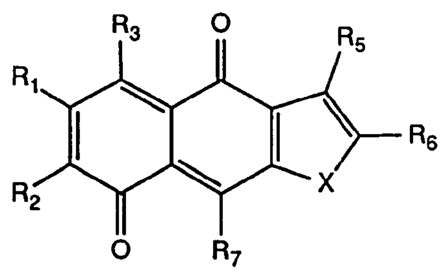

(VI),

worin X und $R_1$ bis $R_7$ die zuvor angegebenen Bedeutungen haben, einschliesslich der Bevorzugungen.

Die Verbindungen der Formel V können nach der Bestrahlung von Lösungen der Verbindungen der Formel I oder II durch Entfernen des Lösungsmittels erhalten und gegebenenfalls nach üblichen Methoden gereinigt werden.

Die Farbänderung ist reversibel. Bei erneuter Bestrahlung mit Licht einer Wellenlänge von etwa 450 bis 650 nm erhält man wieder die ursprüngliche Farbe (Rückbildung der Parachinonstruktur). Besonders vorteilhaft ist, dass dieser Vorgang mehrfach wiederholt werden kann. Die Stabilität der photochemischen Hin- und Rückreaktion ist überraschend hoch und die Ermüdung selbst an Luft oder in Substraten entsprechend gering. So werden bei mehr als 20 Zyklen praktisch keine Veränderungen beobachtet. Als vorteilhaft ist auch anzusehen, dass die zur photochemischen Umwandlung benötigte Lichtabsorption im Bereich der Wellenlänge handelsüblicher Laser liegt.

Ein weiterer Gegenstand der Erfindung ist die Verwendung von Verbindungen der Formeln I oder II beziehungsweise V oder VI als reversible photochrome Systeme zur Kontrastbildung oder Lichtabsorption.

Die Verbindungen der Formel I oder II beziehungsweise V oder VI können als Farbindikatoren in photopolymerisierbaren Systemen verwendet werden. So ist es möglich, belichtete Produkte (z.B. Schutzschichten, Druckplatten, Offsetdruckplatten, gedruckte Schaltungen, Lötstoppmasken) zu markieren und von unbelichteten Produkten zu unterscheiden, und ferner bei einer Produktekontrolle fehlerhaft belichtete Produkte vor oder nach der Entwicklung auszusondern.

Die Verbindungen der Formeln I und II oder V und VI können auch als solche, in Lösung oder in Polymeren eingearbeitet, als Photofarbindikatoren oder als Photoschaltelemente verwendet werden.

Die Verbindungen der Formel I und II beziehungsweise V und VI können auch in organischen oder anorganischen Gläsern als photoschaltbare Farbfilter verwendet werden, z.B. in Gläsern für Sonnenbrillen, Kontaktlinsen, Fenstern und Spiegeln.

Ein weiterer Gegenstand der Erfindung ist eine strahlungsempfindliche Zusammensetzung, enthaltend

a) ein strahlungsempfindliches organisches Material, und

b) mindestens eine Verbindung der Formeln I oder II, beziehungsweise V oder VI oder Mischungen davon.

Die Verbindungen der Formel I oder II und V oder VI oder Mischungen davon können in einer Menge von 0,001 bis 20 Gew.-%, besonders 0,001 bis 10 Gew.-% und insbesondere 0,01 bis 5 Gew.-% enthalten sein, bezogen auf die Komponente a).

Strahlungsempfindliche und damit auch photostrukturierbare Materialien sind bekannt. Es kann sich um Positiv- oder Negativsysteme handeln. Solche Materialien sind z.B. von G. E. Green et al. in J. Macromol. Sci.; Revs. Macromol. und Chem., C21(2), 187-273 (1981 bis 1982) und von G.A. Delzenne in Adv. Photochem., 11, S. 1-103 (1979) beschrieben worden.

Vorzugsweise handelt es sich bei dem strahlungempfindlichen organischen Material um a1) eine nichtflüchtige monomere, oligomere oder polymere Substanz mit photopolymerisierbaren oder photodimerisierbaren ethylenisch ungesättigten Gruppen, a2) um ein kationisch härtbares System oder a3) um photovernetzbare Polyimide.

Photopolymerisierbare Substanzen sind z.B. Acryl- und besonders Methacrylsäureester von Polyolen, z.B. Ethylenglykol, Propandiol, Butandiol, Hexandiol, Di(hydroxymethyl)-cyclohexan, Polyoxyalkylendiole wie z.B. Di-, Tri- oder Tetraethylenglykol, Di- oder Tri-1,2-propylenglykol, Trimethylolmethan, -ethan oder -propan und Pentaerythrit, die alleine, in Mischungen und in Abmischung mit Bindemitteln verwendet werden können.

Photodimerisierbare Substanzen sind z.B. Homo- und Copolymere, die Zimtsäuregruppen oder substituierte Maleinimidylverbindungen in Seitengruppen oder Chalkongruppen in der Polymerkette enthalten.

Bevorzugt sind solche Zusammensetzungen, worin Komponente a1) ein Homo- oder Copolymer von Acryl-, Methacryl- oder Maleinsäureestern ist, deren Estergruppen einen Rest der Formel

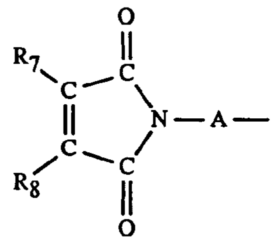

enthalten, worin A für unsubstituiertes oder mit Hydroxyl substituiertes lineares oder verzweigtes $C_2$-$C_{12}$-Alkylen, Cyclohexylen oder Phenylen steht, und $R_7$ und $R_8$ unabhängig voneinander Cl, Br, Phenyl oder $C_1$-$C_4$-Alkyl bedeuten, oder $R_7$ und $R_8$ zusammen Trimethylen, Tetramethylen oder

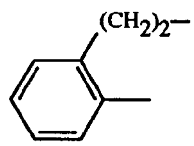

bedeuten. Solche Polymere sind z.B. in der US-A-4 193 927 beschrieben.

Die photopolymerisierbaren oder photodimerisierbaren Substanzen können weitere für die Verarbeitung oder Anwendung übliche Additive enthalten, sowie zusätzlich Photoinitiatoren oder Photosensibilisatoren.

Bei den kationisch härtbaren Systemen handelt es sich bevorzugt um Epoxidverbindungen mit mindestens 2 Epoxidgruppen im Molekül, denen ein Photoinitiator einverleibt ist. Geeignete Photoinitiatoren sind z.B. Cyclopentadienyl-Aren-Metallsalze, Cyclopentadienylmetallcarbnoyl-salze und Oniumsalze, die z.B. in den zuvor erwähnten Publikationen beschrieben sind. Die härtbaren Systeme können für die Verarbeitung und Anwendung übliche Zusatzstoffe enthalten.

Photoempfindliche Polyimide sind z.B. in der DE-A-1 962 588, EP-A-0 132 221, EP-A-0 134 752, EP-A-0 162 017 und EP-A-0 182 745 beschrieben.

Die erfindungsgemässe Zusammensetzung wird nach bekannten Methoden als Schicht auf Substrate aufgebracht und entweder durch flächige Bestrahlung eine Schutzschicht oder durch Bestrahlung unter einer Photomaske oder durch ortsaufgelöste Bestrahlung mittels eines geführten Laserstrahls oder durch holographische Verfahren und nachfolgende Entwicklung ein Reliefbild erzeugt.

Ein weiterer Gegenstand der Erfindung ist eine Zusammensetzung, enthaltend

a) ein farbloses organisches Lösungsmittel, ein Polymer oder ein organisches Glas oder ein Verbundglas, und

b) gelöst, eingemischt oder als Schicht auf mindestens einer Oberfläche eine Verbindung der Formel I oder II beziehungsweise V oder VI oder Mischungen davon. Die Komponente b) ist bevorzugt in einer Menge von 0,001 bis 20 Gew.-%, besonders 0,001 bis 10 Gew.-% und insbesondere 0,01 bis 5 Gew.-% enthalten, bezogen auf Komponente a). Organische Lösungen können zur Beschichtung von anderen Substanzen verwendet werden, z.B. festen Substraten wie zum Beispiel anorganischen Gläsern, die dann als photoschaltbare Substrate verwendet werden können. Die Verbindungen der Formel I oder II beziehungsweise V oder VI können auch auf Substrate aufsublimiert werden. Die beschichteten Substrate konnen mit einer Schutzschicht aus z.B. transparenten Polymeren versehen werden. Feste Substrate können auch mit Zusammensetzungen beschichtet werden, die ein Polymer und mindstens eine Verbindung der Formeln I oder II beziehungsweise V oder VI enthalten. Geeignete Lösungsmittel sind z.B. Kohlenwasserstoffe, halogenierte Kohlenwasserstoffe, Ketone, Carbonsäureester und Lactone, N-alkylierte Säureamide und Lactame, Alkanole und Ether.

Geeignete Polymere sind z.B. Duroplaste, Thermoplaste und strukturell vernetzte Polymere. Die Polymeren sind bevorzugt transparent. Solche Polymere und organische Gläser sind dem Fachmann geläufig. Die Einarbeitung der erfindungsgemässen Verbindungen erfolgt nach üblichen Methoden, z.B. mit Lösungsverfahren und Entfernen des Lösungsmittels, Kalandrieren oder Extrusion. Die erfindungsgemässen Verbindungen können den Substraten auch vor, während oder nach deren Herstellung einverleibt werden.

Ein weiterer Gegenstand der Erfindung ist ein Verfahren zur Herstellung von gefarbten Materialien unter Einwirkung von Licht, dadurch gekennzeichnet, dass man dem Material eine Verbindung der Formel I oder II beziehungsweise V odr VI einverleibt und darauf das Material mit Licht bestrahlt.

Ferner ist ein Gegenstand der Erfindung die Verwendung von Verbindungen der Formel I oder II beziehungsweise V oder VI als Farbindikatoren oder photoschaltbare Farbfilter bei Lichteinwirkung.

Ein weiterer Gegenstand der Erfindung ist die Verwendung der Verbindung der Formel I oder II beziehungsweise V oder VI zur reversiblen optischen Speicherung von Information, wobei die Information in einer die Verbindung enthaltenden, speicheraktiven Schicht mit Licht, bevorzugt Laserlicht, eingeschrieben wird. Die eingeschriebene Information kann mit bevorzugt Laserlicht wieder gelöscht werden, so dass ein zyklisches Einschreiben und Löschen möglich ist.

Zur Herstellung einer speicheraktiven Schicht kann die Verbindung I oder II beziehungsweise V oder VI nach zuvor beschriebenen Verfahren in einer transparenten Matrix gelöst und in dünner Schicht auf ein ebenes Substrat aufgebracht werden. Die Dicke der speicheraktiven Schicht beträgt ca. 0,1-100 µm, bevorzugt 0,3-3 µm.

Geeignete Träger sind zum Beispiel Metalle, Metallegierungen, Gläser, Mineralien, Keramiken und duroplastische oder thermoplastische Kunststoffe. Der Träger kann eine Dikke von 0,01 mm bis 1 cm, bevorzugt 0,1 mm bis 0,5 cm aufweisen. Bevorzugte Träger sind Gläser und homo- oder kopolymere Kunststoffe. Geeignete Kunststoffe sind zum Beispiel thermoplastische Polycarbonate, Polyamide, Polyester, Polyacrylate und Polymethacrylate, Polyurethane, Polyolefine, Polyvinylchlorid, Polyvinylidenfluorid, Polyimide, duroplastische Polyester und Epoxidharze.

Auf dem Träger können ein oder mehrere, zum Beispiel 1 bis 10, bevorzugt 1 bis 5 und besonders bevorzugt 1 bis 3 Schichten aus gleichen oder verschiedenen Verbindungen der Formel I aufgebracht sein. Die Anzahl der Schichten und weiterer Schichten richtet sich hauptsächlich nach der optischen Dichte des Schichtaufbaus, die bei der zur Aufzeichnung verwendeten Wellenlänge noch eine ausreichende Transmission gewährleisten muss.

Auf der speicheraktiven Schicht oder auf dem Träger kann eine Reflexionsschicht aufgebracht sein, die eine Dicke von zum Beispiel 100 bis 5000 Å, bevorzugt 100 bis 3000 Å und besonders von 300 bis 1500 Å aufweisen kann. Als reflektierendes Material eignen sich besonders Metalle, die die zur Aufzeichnung und Wiedergabe verwendete Laserstrahlung gut reflektieren. Besonders bevorzugt ist aus Gründen der hohen Reflektivität und leichten Herstellbarkeit eine Reflexionsschicht aus Aluminium oder Gold.

Die je nach Schichtaufbau oberste Schicht, zum Beispiel die Reflexionsschicht, die speicheraktive Schicht oder eine weitere Hilfsschicht wird zweckmässig mit einer Schutzschicht versehen, die eine Dicke von 0,1 bis 100 µm, bevorzugt 0,1 bis 50 µm und besonders bevorzugt 0,5 bis 15 µm aufweisen kann. Als Schutzmaterial eignen sich hauptsächlich Kunststoffe, die in dünner Schicht entweder direkt oder mit Hilfe von Haftschichten auf den Träger oder die oberste Schicht aufgetragen sind. Man wählt zweckmässig mechanisch und thermisch stabile Kunststoffe mit guten Oberflächeneigenschaften, die noch modifiziert, zum Beispiel beschrieben werden können. Es kann sich sowohl um duroplastische wie auch thermoplastische Kunststoffe handeln. Bevorzugt sind strahlungsgehärtete (zum Beispiel mit UV-Strahlung) Schutzschichten, die besonders einfach und wirtschaftlich herstellbar sind. Strahlungshärtbare Materialien sind in grosser Vielzahl bekannt. Beispiele für strahlungshärtbare Monomere und Oligomere sind Acrylate und Methacrylate von Diolen, Triolen und Tetrolen, Polyimide aus aromatischen Tetracarbonsäuren und aromatischen Diaminen mit $C_1$-$C_4$-Alkylgruppen in mindestens zwei Orthostellungen der Aminogruppen, und Oligomere mit Dialkyl-, zum Beispiel Dimethylmaleinimidylgruppen. Spezifische Beispiele sind UV-vernetzbare Polymere auf der Basis von Polyacrylaten wie zum Beispiel RENGOLUX® RZ 3200/003 oder 3203/001 der Firma Morton International-Dr. Renger.

Geeignete Beschichtungsverfahren sind zum Beispiel Tauchen, Giessen, Streichen, Rakeln, Schleudergiessen und Aufdampfverfahren, die im Hochvakuum durchgeführt werden. Bei der Anwendung von zum Beispiel Giessverfahren werden im allgemeinen Lösungen in organischen Lösungsmitteln verwendet, die noch zusätzlich ein Bindemittel enthalten können. Bei der Verwendung von Lösungsmitteln ist darauf zu achten, dass die verwendeten Träger gegen diese Lösungsmittel unempfindlich sind. Bevorzugt werden alle Schichten mittels Aufdampfverfahren besonders im Vakuum hergestellt. Geeignete Beschichtungsverfahren sind zum Beispiel in der EP-A-0 401 791 beschrieben.

Der Aufbau des erfindungsggemässen Aufzeichnungsmaterials richtet sich hauptsächlich nach der

Auslesemethode; bekannte Funktionsprinzipien sind die Messung der Veränderung der Transmission oder der Reflexion. Wenn das Aufzeichnungsmaterial gemäss der Veränderung der Lichttransmission aufgebaut wird, kommt zum Beispiel folgende Struktur in Frage: Transparenter Träger/Aufzeichnungsschicht (gegebenenfalls mehrschichtig) und falls zweckmässig, transparente Schutzschicht. Das Licht zur Aufzeichnung sowie zum Auslesen kann entweder von der Trägerseite oder der Aufzeichnungsschicht bzw. gegebenenfalls der Schutzschichtseite eingestrahlt werden, wobei sich der Lichtdetektor immer auf der Gegenseite befindet.

Wenn das Aufzeichnungsmaterial gemäss der Veränderung der Reflexion aufgebaut wird, so können zum Beispiel folgende andere Strukturen zur Anwendung kommen: Transparenter Träger/Aufzeichnungsschicht (gegebenenfalls mehrschichig)/Reflexionsschicht und falls zweckmässig, Schutzschicht (nicht unbedingt transparent), oder Träger (nicht unbedingt transparent)/Reflexionsschicht/Aufzeichnungsschicht und falls zweckmässig transparente Schutzschicht. Im ersten Fall wird das Licht von der Trägerseite eingestrahlt, während im letzen Falls die Strahlung von der Aufzeichnungsschicht- bzw. gegebenenfalls von der Schutzschichtseite einfällt. In beiden Fällen befindet sich der Lichtdetektor auf gleicher Seite wie die Lichtquelle. Der ersterwähnte Aufbau des erfindungsgemäss zu verwendenden Aufzeichnungsmaterials ist im allgemeinen bevorzugt.

Das Einschreiben der Information kann durch gerasterte, holographische oder photographische Belichtung der speicheraktiven Schicht mit spektralem, bevorzugt kohärentem (Laser-)Licht im Wellenlängenbereich 440-650 nm, bevorzugt 480-600 nm erfolgen.

Das Auslesen kann mit reduzierter Strahlungsleistung bei der Einschreibwellenlänge über die örtlich geänderte Transmission, Reflexion, Brechung oder Fluoreszenz der speicheraktiven Schicht erfolgen.

Das Löschen kann durch punktförmige oder flächige Belichtung der speicheraktiven, die Verbindungen der Formeln I, II und /oder V, VI enthaltenden Schicht im Wellenbereich 300-450 nm, bevorzugt 300-400 nm erfolgen.

Ein Vorteil der erfindungsgemässen Verwendung ist, dass die zum Einschreiben, Auslesen und Löschen erforderlichen Wellenlängen im Bereich handelsüblicher Laser liegen (z.B. Argonionenlaser: 488/514 nm bzw. 351/363 nm; Neodym-YAG-Laser: 532 nm bzw. 355 nm; XeF-Excimerlaser: 351 nm; HeCd-Laser: 325 und 442 nm, bei Frequenzverdopplung und Verdreifachung).

Ein weiterer Vorteil ist der hohe erreichbare Kontrast der Absorption zwischen dem beschriebenen und dem gelöschten Zustand im Bereich 450-650 nm und die damit verbundene grosse Dynamik der speicheraktiven Schicht.

Ein anderer Vorteil ist, dass die Quantenausbeute beim Einschreiben relativ niedrig ist und damit die Gefahr eines Ueberschreibens beim Auslesen stark reduziert wird.

Vorteilhaft ist auch umgekehrt, dass die Quantenausbeute beim Löschvorgang relativ hoch ist und damit ein rasches grossflächiges Löschen ermöglicht wird.

Ein weiterer Vorteil ist, dass die Verbindung beim Auslesen fluoresziert und somit eine hochsensitive Detektion des Speicherzustands über die Fluoreszenz ermöglicht. Dass die zum Auslesen eingestrahlte Energie wesentlich über die Fluoreszenz und nicht thermisch dissipiert, wirkt zudem einer unerwünschten Erwärmung der speicheraktiven Schicht entgegen.

Ein weiterer Vorteil ist die hohe Photostabilität der Verbindung und die dadurch erreichbare hohe Zahl von Schreib-/Löschzyklen.

Schliesslich besteht als weiterer Vorteil die Möglichkeit einer zyklischen Datenauffrischung durch Zumischen eines geeigneten Quantums Licht der Löschwellenlänge während des Auslesens.

Die nachfolgenden Beispiele erläutern die Erfindung.

A) Herstellung von Ausgangsverbindungen

Beispiel A1: Herstellung von 5,6,7,8-Tetrachlor-1-methyl-benz[f]indol-4,9-dion.

a)3,4,5,6-Tetrachlor-2-(pyrrol-2-ylcarbonyl)benzoesäure

Zu einer Mischung von 6,9 g (28 mmol) Magnesium in 70 ml Anisol werden unter Argon 23,3 ml (280 mmol) Ethyliodid zugetropft und eine Stunde auf 65°C erhitzt. Man tropft in die auf 0 - 5°C gekühlte Mischung eine Lösung von 19,6 g (280 mmol) Pyrrol in 140 ml Anisol und hält anschließend je 15 min bei Raumtemperatur und bei 50°C. Bei 30°C werden 57,2 g (200 mmol) Tetrachlorphthalsäureanhydrid in 70 ml Anisol schnell zugegeben und eine Stunde auf 100°C erhitzt. Nach dem Abkühlen wird mit 210 ml verdünnter Essigsäure (2:5) angesäuert und die organische Phase abdekantiert, letztere mit 10% NaOH versetzt und das dabei ausfallende Natriumsalz der Säure abfiltriert. Das Filtrat wird mit wässriger HCl angesäuert, wodurch 14,8 g (21 %) der Säure isoliert werden können.

b) 3,4,5,6-Tetrachlor-2-(1-methyl-pyrrol-2-ylcarbonyl)benzoesäure

4 g (11 mmol) des Natriumsalzes der 3,4,5,6-Tetrachlor-2-(pyrrol-2-ylcarbonyl)benzoesäure, 43 ml 35%-ige KOH und 3,3 ml (54 mmol) Methyliodid werden 20 h auf 45°C erwärmt und der entstehende Niederschlag bei Raumtemperatur abfiltriert, mit Wasser gewaschen und getrocknet. Man erhält 3 g (77 %) der Benzoesäure, Schmelzpunkt>270°C.

c) Herstellung der Titelverbindung

Eine Mischung aus 2,8 g (8 mmol) 3,4,5,6-Tetrachlor-2-(1-methyl-pyrrol-2-ylcarbonyl)benzoesäure und 8,4 g (63 mmol) $AlCl_3$ werden in 42 ml Nitrobenzol 12 h auf 120°C erhitzt. Das Reaktionsgemisch wird in 2N HCl gegossen, die organische Phase in einer Mischung aus Toluol und Tetrahydrofuran (THF, 1:1) aufgenommen, mit 10%-iger wässriger $NaHCO_3$-Lösung und Wasser gewaschen, getrocknet und die Lösungsmittel abdestilliert. Die chromatographische Reinigung (Kieselgel, Methylenchlorid) ergibt 260 mg (10 %) des Chinons, Schmelzpunkt 238 - 240°C.

<u>Beispiel A2</u>: Herstellung von 7,10-Dichlor-benzo[b]naphtho[2,3-d]thiophen-6,11-dion.

a) 2-(Benzthiophen-2-ylcarbonyl)-3,6-dichlorbenzoesäure

Zu einer unter Argon gehaltenen Lösung von 17,3 ml (27,7 mmol) n-Butyllithium (1,6 M in Hexan) und 20 ml absolutem Diethylether wird bei 0 - 10°C eine Lösung von 3,4 g (25,3 mmol) Benzthiophen in 15 ml absolutem Diethylether zugetropft. Man rührt 30 min bei Raumtemperatur, dann 1 h unter Rückfluß. Diese Lösung gibt man bei 0 - 10°C zu einer Lösung von 5 g (23 mmol) 3,6-Dichlorphthalsäureanhydrid in 25 ml absolutem Diethylether und 45 ml absolutem THF und rührt 2,5 h bei 0 - 5°C und 12 h bei Raumtemperatur. Es wird hydrolysiert, die organische Phase abgetrennt, mit Wasser gewaschen, mit $Na_2SO_4$ getrocknet und die Lösungsmittel am Rotationsverdampfer entfernt. Der Rückstand wird säulenchromatographisch gereinigt (Kieselgel, Essigsäureethylester). Man erhält 5,1 g (63 %), Zersetzungspunkt 120°C.

b) Herstellung der Titelverbindung

1,5 g (4,3 mmol) 2-(Benzthiophen-2-ylcarbonyl)-3,6-dichlorbenzoesäure werden in 20 ml Nitrobenzol mit 3,9 g (29,2 mmol) wasserfreiem $AlCl_3$ 2 h auf 135°C erhitzt. Das Reaktionsgemisch wird mit 2 N HCl hydrolysiert und mit Toluol/THF (1:1) extrahiert. Die organische Phase wird mit 10%-iger wässriger $NaHCO_3$-Lösung gewaschen, mit $Na_2SO_4$ getrocknet und eingeengt. Man reinigt den Rückstand säulenchromatographisch (Kieselgel, Toluol/Hexan 1:1) und erhält 0,73 g (51 %), Schmelzpunkt>250°C.

<u>Beispiel A3</u>: Herstellung eines Isomerengemisches aus 5,10-Dichlor-anthra[2,3-b]thio-phen-4,11-dion und 4,11-Dichlor-anthra[2,3-b]thiophen-5,10-dion.

a) Herstellung von 5,10-Dihydroxy-anthra[2,3-b]thiophen-4,11-dion.

1 g (6,5 mmol) Thiophen-2,3-dicarbonsäureanhydrid, 0,7 g (4,4 mmol) 1,4-Dihydroxynaphthalin, 0,45 g (6,5 mmol) Borsäureanhydrid, 6 mg Natriumdithionit und 2,5 ml Tetramethylensulfon werden gemischt und 2 h auf 220°C erhitzt. Nachdem das Gemisch wieder 110°C erreicht hat, wird mit 10 ml heißem Wasser versetzt und der Niederschlag abgesaugt. Das Produkt wird mit Wasser gewaschen, getrocknet und säulenchromatographisch (Kieselgel, Methylenchlorid) gereinigt. Man erhält 0,29 g (22 %), Schmelzpunkt 215 - 220°C.

b) Herstellung der Titelverbindungen.

295 mg (1 mmol) 5,10-Dihydroxy-anthra[2,3-b]thiophen-4,11-dion werden 3 Tage in 5 ml Phosphoroxychlorid bei 120°C gerührt, die Lösung auf Eiswasser gegossen und der schwarze Niederschlag abfiltriert. Nach dem Waschen mit Wasser und Methanol und dem Trocknen erhält man 225 mg (68 %) der Chinone, Schmelzpunkt 226 - 228°C.

<u>Beispiel A4</u>: Herstellung von 5-Benzyl-10-chlor-benzo[b]carbazol-6,11-dion.

Eine Lösung von 1 g (4,7 mmol) 3-Chlorbenzoesäurediethylamid in 132 ml absolutem Diethylether/THF (10:1) wird unter Inertgas auf -72°C gekühlt und 0,7 ml (4,7 mmol) Tetramethylendiamin zugegeben. Nach der Zugabe von 3,4 ml 1,4 M sec-Butyllithium in Hexan wird 2 h bei dieser Temperatur gehalten und 1,1 g (4,7 mmol) 1-Benzylindol-3-carbaldehyd in 48 ml Diethylether/THF (5:1) zugetropft. Nach 1,5 h lässt man langsam auf -20°C erwärmen, kühlt wieder auf -72°C, gibt erneut 4,25 ml 1,4 M sec-Butyllithium zu und lässt über Nacht auf Raumtemperatur erwärmen. Die organische Phase wird mit Wasser gewaschen, getrocknet und am Rotationsverdampfer eingeengt. Nach säulenchromatographischer Reinigung (Kieselgel, Toluol) werden 260 mg (15 %) des Chinons isoliert, Schmelzpunkt 195 - 200°C.

Beispiel A5: Herstellung von 10-Chlor-5-phenyl-benzo[b]carbazol-6,11-dion.

Analog Beispiel A4 erhält man aus 1 g 1-Phenylindol-3-carbaldehyd 60 mg (3,5 %) 10-Chlor-5-phenyl-benzo[b]carbazol-6,11-dion, Schmelzpunkt>280°C.

Beispiel A6: Herstellung von 1-Benzyl-5-chlor-benz[f]indol-4,9-dion.

a) 7-Chlor-3-(1-benzyl-pyrrol-2-yl)phthalid

Eine Lösung von 6,3 g (30 mmol) 2-Chlorbenzoesäurediethylamid in 825 ml absolutem Diethylether/THF (10:1) wird unter Inertgas auf -72°C gekühlt und 4,5 ml (30 mmol) Tetramethylendiamin zugegeben. Nach der Zugabe von 24 ml 1,4 M sec-Butyllithium in Hexan wird 2,5 h bei dieser Temperatur gehalten und 5,5 g (30 mmol) 1-Benzylpyrrol-2-carbaldehyd in 5 ml Diethylether zugetropft. Nach 2 h lässt man langsam auf Raumtemperatur kommen. Die organische Phase wird mit Wasser gewaschen, getrocknet und am Rotationsverdampfer eingeengt. Der Rückstand wird in Chloroform gelöst, 8 g Kieselgel zugegeben und 17 h am Rückfluß erhitzt. Das abgekühlte Reaktionsgemisch wird filtriert und eingeengt. Nach säulenchromatographischer Reinigung (Kieselgel, Methylenchlorid) werden 4,4 g (42 %) des Phthalids isoliert, Schmelzpunkt 134 - 136°C.

b) 6-Chlor-2-(1-benzyl-pyrrol-2-ylmethyl)benzoesäure

3 g (9,3 mmol) 7-Chlor-3-(1-benzyl-pyrrol-2-yl)phthalid in 150 ml Pyridin werden mit 670 ml 15%-iger wässriger KOH-Lösung, 500 ml Wasser und 24 g mit Kupfer aktiviertem Zink 24 h am Rückfluß erhitzt. Der Niederschlag wird abgesaugt, das Filtrat mit konzentrierter HCl auf pH 5 - 6 eingestellt und mit $CH_2Cl_2$ extrahiert. Man trocknet die organische Phase mit $Na_2SO_4$ und engt ein. Der Rückstand wird säulenchromatographisch gereinigt (Kieselgel, $CH_2Cl_2$/Hexan 2:1), man erhält 0,48 g (16 %).

c) Herstellung der Titelverbindung

360 mg (1,1 mmol) 6-Chlor-2-(1-benzyl-pyrrol-2-ylmethyl)benzoesäure werden in 4 ml $CH_2Cl_2$ suspendiert, 1,6 ml (1,1 mmol) Trifluoressigsäureanhydrid zugegeben und 6 h bei Raumtemperatur gerührt. Man neutralisiert die organische Phase mit 10%-iger wässriger $NaHCO_3$-Lösung, wäscht mit Wasser, trocknet mit $Na_2SO_4$ und engt ein. Der Rückstand wird in 4 ml 40%-igem Triton® B in Methanol und 20 ml Methanol gelöst und mehrere Stunden Luft durch die Lösung geleitet. Danach versetzt man mit $CH_2Cl_2$, wäscht die organische Phase mit 2 N HCl und Wasser neutral, trocknet mit $Na_2SO_4$ und engt ein. Der Rückstand wird säulenchromatographisch gereinigt (Kieselgel, $CH_2Cl_2$/Hexan 2:1). Man erhält 20 mg (6 %) des Produktes.

Beispiel A7: Herstellung von N-Phenyl-5-chlor-benz[f]indol-4,9-dion.

Analog zu Beispiel A6, aber unter Verwendung von 1-Phenylpyrrol-2-carbaldehyd, werden 56% 7-Chlor-3-(1-phenyl-pyrrol-2-yl)phthalid erhalten. Dieses wird analog zu Beispiel A6b) zur 6-Chlor-2-(1-phenyl-pyrrol-2-ylmethyl)benzoesäure (5% Ausbeute) und dann analog zu Beispiel A6c) zur Titelverbindung (30% Ausbeute) umgesetzt.

Beispiel A8: Herstellung eines Isomerengemisches von 3-Cyano-5- und -8-nitro-2-phenyl-naphtho[2,3-b]furan4,9-dion.

Zu einer Suspension von 4 g (14,7 mmol) 2,3-Dichlor-5-nitronaphthochinon und 3,2 g (22 mmol) Benzoylacetonitril gibt man 40 ml Pyridin und erhitzt 4,5 h am Rückfluß. Das Reaktionsgemisch wird über Nacht auf 5°C gekühlt und der braune Niederschlag abgesaugt, mit Ethanol und Diethylether gewaschen und getrocknet. Die chromatographische Reinigung (Kieselgel, Toluol/Hexan 2:1) ergibt 660 mg (13 %) der Titelverbindungen, Schmelzpunkt>300°C.

Beispiel A9: Herstellung eines Isomerengemisches von 3-Acetyl-2-methyl-5- und -8-nitro-naphtho[2,3-b]furan-4,9-dion.

Zu einer Suspension von 3 g (11 mmol) 2,3-Dichlor-5-nitronaphthochinon in 100 ml absolutem Ethanol gibt man eine Lösung von 2,6 g (22 mmol) Natriumacetylacetonat in 50 ml absolutem Ethanol, rührt 40 min und filtriert. Das Filtrat wird mit 2 N HCl angesäuert und mit $CH_2Cl_2$ extrahiert. Die organische Phase wird mit Wasser gewaschen, über $Na_2SO_4$ getrocknet und eingeengt. Der Rückstand wird säulenchromatographisch gereinigt (Kieselgel, $CH_2Cl_2$/Hexan 1:1). Man erhält 3,4 g (92 %). Man löst die Substanz in 150 ml Ethanol, gibt 30 ml Tributylamin zu und erhitzt 1 h am Rückfluß. Das Reaktionsgemisch wird in verdünnte HCl gegossen und

mit $CH_2Cl_2$ extrahiert. Die organische Phase wird mit verdünnter HCl, gesättigter wässriger NaCl-Lösung und Wasser gewaschen, über $Na_2SO_4$ getrocknet und eingeengt. Der Rückstand wird durch Säulenchromatographie ge- reinigt (Kieselgel, $CH_2Cl_2$/Hexan 1:1) und ergibt 2,2 g (73 %) des Produktes. Schmelzpunkt 198 - 202°C.

Beispiel A10: Herstellung eines Isomerengemisches von 2-Carboxyethyl-5-ethyl-7- und -10-nitro-benzo[b]carbazol-6,-dion.

a) Isomerengemisch von 2-(4-Carboxyethyl-anilino)-5- und -8-nitro-1,4-naphthochinon

3 g (11 mmol) 2,3-Dichlor-5-nitronaphthochinon und 1,82 g (11 mmol) p-Aminobenzoesäureethylester werden in 100 ml Pyridin 16 h am Rückfluss erhitzt. Nachdem man das Reaktionsgemisch in 2 N HCl gegossen hat, extrahiert man mit $CH_2Cl_2$, wäscht die organische Phase mit 2 N HCl, trocknet über $Na_2SO_4$ und engt ein. Der Rückstand wird durch Säulenchromatographie gereinigt (Kieselgel, $CH_2Cl_2$). Man erhält 830 mg (21 %) Produkt.

b) Isomerengemisch von 2-Carboxyethyl-7- und -10-nitro-benzo[b]carbazol-6,11-dion

1,4 g (3,8 mmol) 2-(4-Carboxyethyl-anilino)-5- und -8-nitro-1,4-naphthochinon, 0,86 g (3,8 mmol) Palladium(II)acetat und 0,41 g (3,8 mmol) p-Benzochinon werden in 25 ml Eisessig 7 h bei 110°C gerührt, dann in 350 ml Wasser gegeben und das Produkt mit $CH_2Cl_2$ extrahiert. Die organische Phase wird mit Wasser gewaschen, mit $Na_2SO_4$ getrocknet und eingeengt. Der dunkelrote Rückstand wird mit $CH_2Cl_2$/Methanol (1:5) verrührt, der zurückbleibende orangefarbene Feststoff mit Methanol gewaschen und getrocknet. Man erhält 213 mg (15 %) Produkt, Schmelzpunkt>300°C.

c) Herstellung der Titelverbindungen

195 mg (0,53 mmol) 2-Carboxyethyl-7- und -10-nitro-benzo[b]carbazol-6,11-dion, 170 mg (1,07 mmol) Ethyliodid und 150 mg (1,07 mmol) Kaliumcarbonat werden in 5 ml DMF 3 h bei 40°C gerührt. Das Reaktionsgemisch wird mit 10-%iger HCl versetzt und das Produkt mit $CH_2Cl_2$ extrahiert. Die organische Phase wird mit gesättigter wässriger NaCl-Lösung gewaschen, mit $Na_2SO_4$ getrocknet und eingeengt. Der Rückstand wird säulenchromatographisch gereinigt (Kieselgel, $CH_2Cl_2$) und ergibt 195 mg (93 %) des Produktes. Schmelzpunkt 271 - 277°C.

Beispiel A11: Herstellung von 5,8-Dichlor-naphtho[2,3-d]thiophen-4,9-dion.

Aus 0,75 g (30,75 mmol) Magnesiumspänen, 36 ml absolutem Diethylether und 4,01 g (24,6 mmol) 2-Bromthiophen wird unter Rühren am Rückfluss eine Suspension von Thiophen-2-magnesiumbromid hergestellt und diese bei 45-50°C zu einer Lösung von 5,34 g (24,6 mmol) 3,6-Dichlor-phthalsäureanhydrid zugetropft. Es wird 90 min am Rückfluss gerührt, dann mit verdünnter Salzsäure hydrolysiert und mit Toluol extrahiert. Die vereinigten organischen Phasen werden über $Na_2SO_4$ getrocknet und danach eingedampft. Das Rohprodukt (7,4 g) wird in 77 ml Nitrobenzol aufgenommen, die Mischung 15,3 g $AlCl_3$ versetzt und während 6,5 h bei 135°C gerührt. Nach dem Abkühlen wird mit 2N wässriger HCl hydrolysiert und daruf mit Tetrahydrofuran/Toluol extrahiert. Die vereinigten organischen Phasen werden mit wässriger $NaHCO_3$-Lösung, Wasser und gesättigter NaCl-Lösung gewaschen, dann über $Na_2SO_4$ getrocknet und darauf eingedampft. Durch Säulenchromatographie an Kieselgel (Methylenchlorid/Hexan 3:2) und Umkristallisation aus Toluol/Hexan erhält man 1,46 g (30%) der Titelverbindung.

B) Herstellung von Aryloxy-substituierten Verbindungen.

Beispiele B1 bis B16:

Die Verbindungen der Beispiele A1 bis A10, Phenol und 210 mg (1,5 mmol) $K_2CO_3$ werden in 2 ml Lösungsmittel 14 h auf 115°C erhitzt. Danach giesst man das Reaktionsgemisch auf verdünnte HCl, saugt den Niederschlag ab, wäscht mit Wasser und wenig Methanol und trocknet den Rückstand im Vakuum. Das Rohprodukt wird säulenchromatographisch gereinigt (Kieselgel, Methylenchlorid) und aus Methylenchlorid/Hexan umkristallisiert. In den Beispielen 1 bis 4, 7 und 9 bis 15 wird Dimethylsulfoxid, in den Beispielen 6 und 8 Phenol und in Beispiel 16 Toluol als Lösungsmittel verwendet. Äquivalente Phenol und Reaktionstemperaturen in °C: B1 (4/100), B2 (2/80), B3 und B4 (1/90), B5 (6/110), B6 (>1, Phenol ist Lösungsmittel/140), B7 (2/120), B8 (>1, Phenol ist Lösungsmittel/120), B9 (2,4/110), B10 (2,5/80), B11 bis B14 (2,5/60), B15 (5/80) und B16 (2,5/110). Weitere Angaben befinden sich in der nachfolgenden Tabelle:

| Beispiel Nr. | Ausgangs-produkt | Reaktionsprodukt | Schmelz-punkt (°C) | Ausbeu-te (%) |
|---|---|---|---|---|
| B1 | A2 | 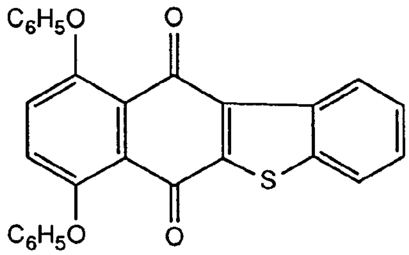 | 213-216 | 30 |

| | | | | |
|---|---|---|---|---|
| B2 | A11 | 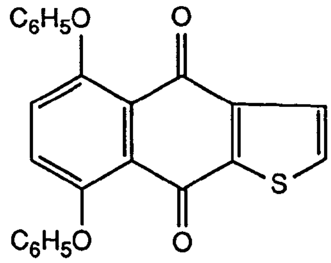 | 200-204 | 44 |
| B3 | A2 | 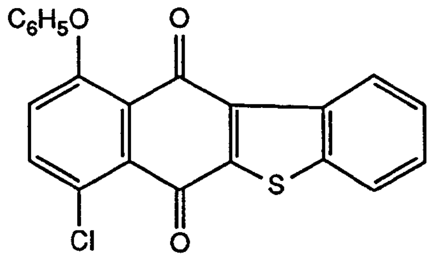 und 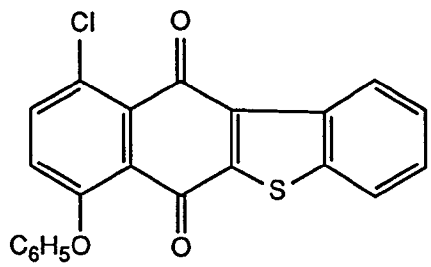 | 210-218 | 47 |
| B4 | A3 | 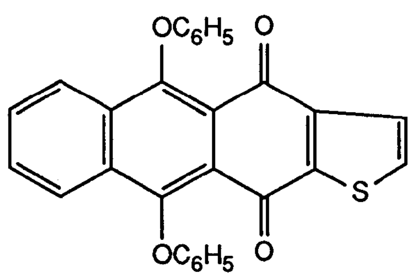 und 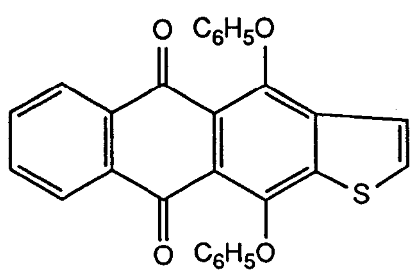 | 240-244 | 79 |

| | | | | |
|---|---|---|---|---|
| B5 | A1 | 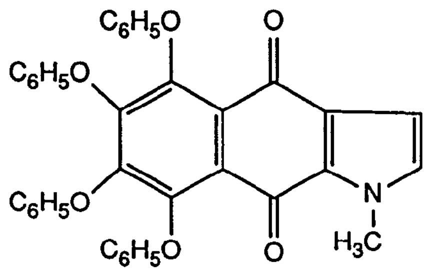 | 186-188 | 30 |
| B6 | A6 | 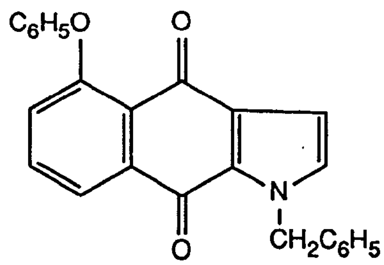 | 130-133 | 53 |
| B7 | A7 | 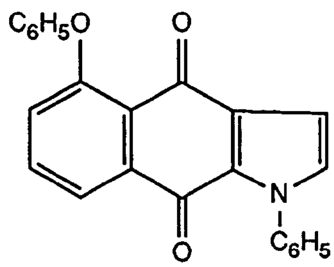 | 165-168 | 10 |
| B8 | A4 | 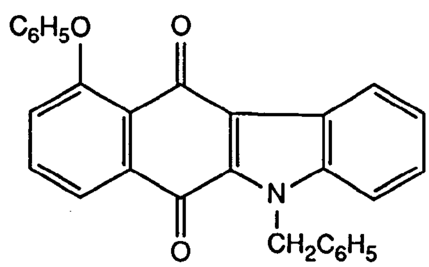 | 211-213 | 36 |
| B9 | A5 | 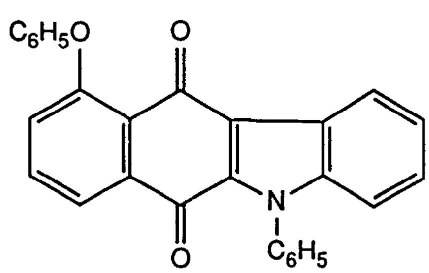 | 229-231 | 33 |

| | | | | |
|---|---|---|---|---|
| B10 | A10 | 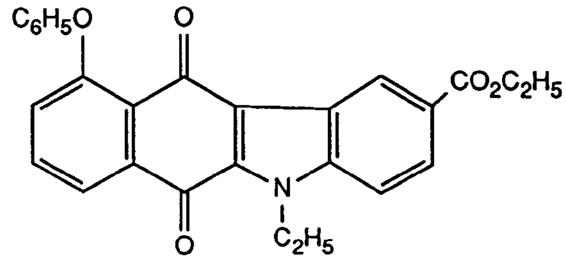 und 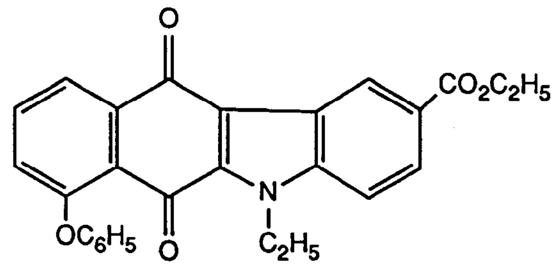 | >260 | 74 |
| B11 | a) | 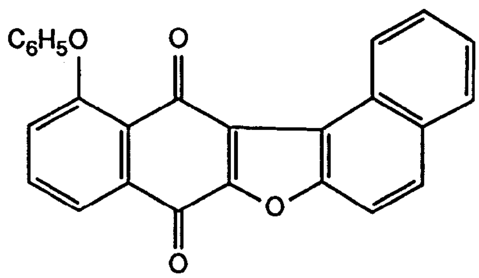 | >290 | 35 |
| B12 | a) | 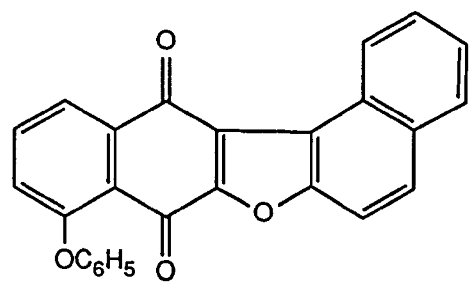 | 256-258 | 17 |
| B13 | a) | 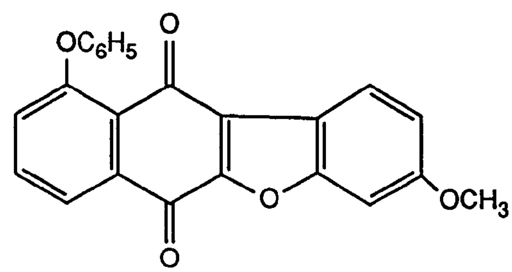 | 245-247 | 28 |

| | | | | |
|---|---|---|---|---|
| B14 | a) | 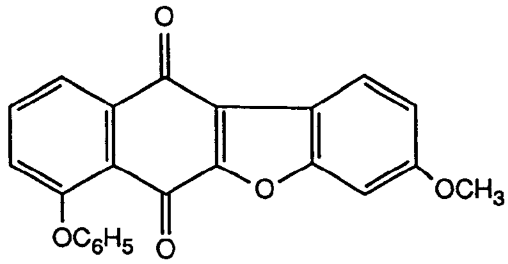 | 283-284 | 43 |
| | | 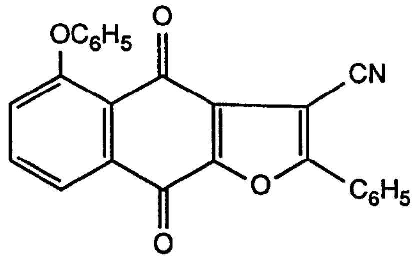 | | |
| B15 | A8 | und | 205-208 | 74 |
| | | 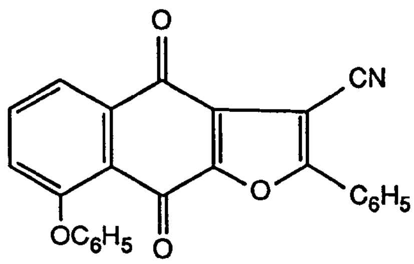 | | |
| | | 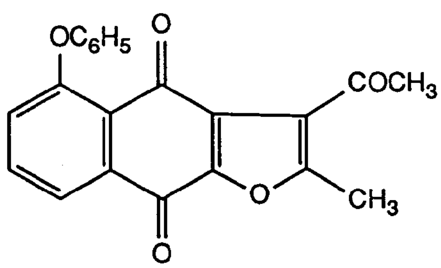 | | |
| B16 | A9 | und | 144-147 | 5 |
| | | 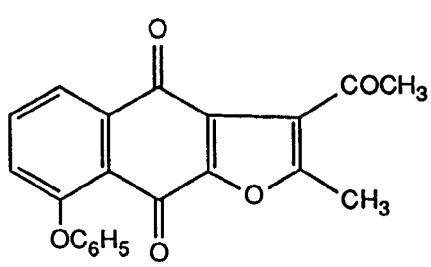 | | |

a) Die entsprechenden Ausgangsverbindungen werden nach Literaturvorschriften hergestellt: J. Org. Chem.

24, S. 1756 (1959) und J. Org. Chem. 26, S. 3152 (1961).

C) Anwendungsbeispiele

Beispiele C1-C16:

Die Verbindungen beziehungsweise Verbindungsgemische der Beispiele B1 bis B16 (Parachinone) werden als Schicht auf einen Glasträger aufgebracht und mit Licht einer Wellenlänge von 350-420 nm bestrahlt, wobei sich die Anachinone B bilden. Wenn diese so erhaltenen Schichten mit den Anachinonen B mit Licht einer Wellenlänge von 450-600 nm bestrahlt, so erhält man wieder Schichten mit den Parachinonen A. In der nachfolgenden Tabelle sind die Absorptionsmaxima in nm der Ausgangsverbindungen [$\lambda_{max}$(A)] B1 bis B16 und der entsprechenden Anachinone [$\lambda_{max}$(B)] der reversiblen photochromen Systeme angegeben:

| Beispiel Nr. | Verbindung Nr. | [$\lambda_{max}$(A)] | [$\lambda_{max}$(B)] |
|---|---|---|---|
| C1 | B1 | 397 | 527 |
| C2 | B2 | 380 | 500 |
| C3 | B3 | 392 | 486 |
| C4 | B4 | 394 | 476 |
| C5 | B5 | 360 | 520 |
| C6 | B6 | 367 | 515 |
| C7 | B7 | 367 | 515 |
| C8 | B8 | 380 | 580 |
| C9 | B9 | 380 | 580 |
| C10 | B10 | 382 | 570 |
| C11 | B11 | 435 | 538 |
| C12 | B12 | 437 | 587 |
| C13 | B13 | 400 | 524 |
| C14 | B14 | 425 | 577 |
| C15 | B15 | 392 | 499 |
| C16 | B16 | 379 | 511 |

Beispiel C17: In 1 g 50°C warmem Ethoxy-bisphenol-A-dimethylacrylat werden 25 mg der Verbindung B14 gelöst, 1 g 50°C warmes Bisphenol-A-diglycidyl-diacrylat und 10 mg Azo-isobutyronitril zugegeben und unter Rühren gemischt bis eine klare, orange Mischung entsteht.

Die Formulierung wird zwischen zwei Glasplatten mit 1 mm-Teflonspacer gegeben und 20 h bei 80°C ausgehärtet.

Die klare, orangefarbene Platte wird teilweise abgedeckt und mit einer 200 W HG-Kurzbogenlampe 2 min durch einen 420 nm-Langpassfilter bestrahlt. Die bestrahlte Zone verfärbt sich blau-grau. Die belichtete Zone lässt sich durch Bestrahlung durch einen 515 nm-Langpassfilter wieder in den ursprünglichen Zustand zurückschalten.

## Patentansprüche

1. Verbindungen der Formeln I und II oder Gemische von Verbindungen der Formel I und Verbindungen der Formel II,

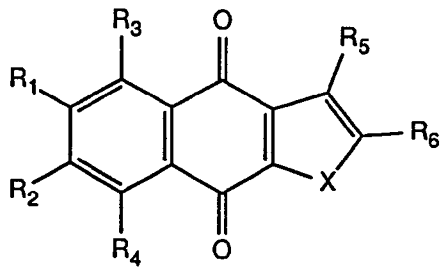

(I),

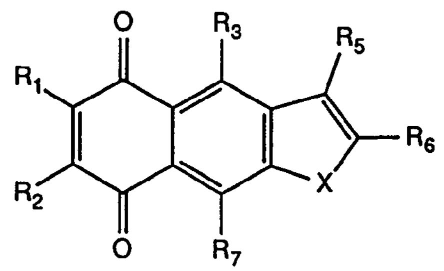

(II),

worin

X fur -O-, -S- oder $-NR_8-$ steht,

$R_1$ und $R_2$ unabhängig voneinander H, $C_1$-$C_4$-Alkyl, $C_1$-$C_4$-Alkoxy, Phenyl, Benzyl, $-OR_9$, -CN, $-CO_2R_{10}$, $-CO-R_{11}$, Halogen, oder $R_1$ und $R_2$ zusammen $-CH=CR_{12}-CR_{13}=CH-$ bedeuten;

$R_3$ die Gruppe $-OR_9$ und $R_4$ H, Halogen oder die Gruppe $-OR_9$ darstellen;

$R_5$ und $R_8$ unabhängig voneinander die gleiche Bedeutung wie $R_1$ und $R_2$ haben oder $R_5$ und $R_6$ zusammen 1,2-Benz-buta-1,3-dien-1,4-diyl bedeuten;

$R_7$ H, Halogen oder die Gruppe $-OR_9$ darstellt;

$R_8$ H, lineares oder verzweigtes $C_1$-$C_{18}$-Alkyl oder unsubstituiertes oder mit $C_1$-$C_4$-Alkyl oder $C_1$-$C_4$-Alkoxy substituiertes Phenyl oder Benzyl bedeutet;

$R_9$ unsubstituiertes oder mit $C_1$-$C_{12}$-Alkyl, $C_1$-$C_{12}$-Alkoxy, $C_1$-$C_{12}$-Alkylthio, $C_1$-$C_{12}$-Alkylsulfinyl, $C_1$-$C_{12}$-Alkylsulfonyl, Phenyl, Benzyl, -CN, $-CF_3$, Halogen, $-CO-R_{11}$ oder $-CO_2R_{10}$ substituiertes $C_6$-$C_{14}$-Aryl darstellt;

$R_{10}$ für H, $C_1$-$C_{18}$-Alkyl, Cyclohexyl, Cyclopentyl, Phenyl, $C_1$-$C_{12}$-Alkylphenyl, Benzyl oder $C_1$-$C_{12}$-Alkylbenzyl steht;

$R_{11}$ H, $C_1$-$C_4$-Alkyl oder Phenyl bedeutet; und

$R_{12}$ und $R_{13}$ unabhängig die Bedeutung von $R_1$ und $R_2$ haben.

2. Verbindungen gemäss Anspruch 1, dadurch gekennzeichnet, dass es sich bei $R_1$ und $R_2$ in der Bedeutung von Alkyl und Alkoxy um Methyl, Ethyl, Methoxy und Ethoxy handelt.

3. Verbindungen gemäss Anspruch 1, dadurch gekennzeichnet, dass es sich bei $R_1$ und $R_2$ in der Bedeutung von Halogen um F, Cl oder Br handelt.

4. Verbindungen gemäss Anspruch 1, dadurch gekennzeichnet, dass $R_1$ und $R_2$ H oder die Gruppe $R_9$, oder $R_1$ und $R_2$ zusammen die Gruppe -CH=CH-CH=CH- bedeuten.

5. Verbindungen gemäss Anspruch 1, dadurch gekennzeichnet, dass $R_4$ und $R_7$ in der Bedeutung von Halogen Cl bedeuten.

6. Verbindungen gemäss Anspruch 1, dadurch gekennzeichnet, dass $R_8$ $C_1$-$C_4$-Alkyl, Phenyl oder Benzyl darstellt.

7. Verbindungen gemäss Anspruch 1, dadurch gekennzeichnet, dass $R_5$ und $R_8$ H, $C_1$- oder $C_2$-Alkyl, $C_1$- oder $C_2$-Alkoxy, -CN, $-CO_2$-($C_1$-$C_4$-Alkyl), -CO-($C_1$-$C_4$-Alkyl), Phenyl, Benzyl, oder $R_8$ und $R_8$ zusammen 1,2-Benz-buta-1,3-dien-1,4-diyl oder die Gruppe $-CH=CR_{12}-CR_{13}=CH-$ bedeuten, worin $R_{12}$ und $R_{13}$ für H, $C_1$- oder $C_2$-Alkyl, $C_1$- oder $C_2$-Alkoxy oder $-CO_2$-($C_1$-$C_4$-Alkyl) stehen.

8. Verbindungen gemass Anspruch 7, dadurch gekennzeichnet, dass eines von $R_{12}$ und $R_{13}$ für H steht.

9. Verbindungen gemäss Anspruch 7, dadurch gekennzeichnet, dass $R_9$ unsubstituiertes oder substituiertes Phenyl darstellt.

10. Verbindungen gemäss Anspruch 1, dadurch gekennzeichnet, dass $R_{10}$ H oder $C_1$-$C_{18}$-Alkyl ist.

11. Verbindungen gemäss Anspruch 1, dadurch gekennzeichnet, dass $R_{11}$ H, Methyl, Ethyl oder Phenyl darstellt.

12. Verbindungen gemäss Anspruch 1, dadurch gekennzeichnet, dass $R_{12}$ und $R_{13}$ für H, $C_1$- oder $C_2$-Alkyl, $C_1$- oder $C_2$-Alkoxy oder $-CO_2-(C_1-C_4\text{-Alkyl})$ stehen.

13. Verbindungen gemäss Anspruch 12, dadurch gekennzeichnet, dass eines von $R_{12}$ und $R_{13}$ für H und das andere von $R_{12}$ und $R_{13}$ für $C_1$- oder $C_2$-Alkyl, $C_1$- oder $C_2$-Alkoxy oder $-CO_2-(C_1-C_4\text{-Alkyl})$ stehen.

14. Verbindungen gemäss den Ansprüchen 12 oder 13, dadurch gekennzeichnet, dass $R_{12}$ und/oder $R_{13}$ für Methoxy, Ethoxy, $-C(O)OCH_3$ oder $-C(O)OC_2H_5$ stehen.

15. Verbindungen gemäss Anspruch 1, dadurch gekennzeichnet, dass $R_1$ und $R_2$ H oder $-OC_6H_5$ oder $R_1$ und $R_2$ zusammen -CH=CH-CH=CH- bedeuten, $R_3$ $-OC_6H_5$ und $R_4$ und $R_7$ H, Cl oder $-OC_6H_5$ darstellen, $R_5$ und $R_5$ unabhängig voneinander H, -CN, Methyl, Ethyl, $-C_6H_5$ oder $-CO-CH_3$ oder $-C(O)-C_2H_5$ oder $R_5$ und $R_6$ zusammen 1,2-Benz-buta-1,3-dien-1,4-diyl oder die Gruppe $-CH=CR_{12}-CR_{13}=CH-$ bedeuten, worin $R_{12}$ und $R_{13}$ unabhängig voneinader H, Methyl, Ethyl, Methoxy, Ethoxy, $-CO_2CH_3$ oder $-CO_2C_2H_5$ sind, und X -O-, -S- oder $-NR_5-$ darstellt, worin $R_5$ $C_1$-$C_4$- Alkyl, Benzyl oder Phenyl ist.

16. Verfahren zur Herstellung von Verbindungen der Formel I und II gemäss Anspruch 1, dadurch gekennzeichnet, dass man eine Verbindung der Formeln III oder IV,

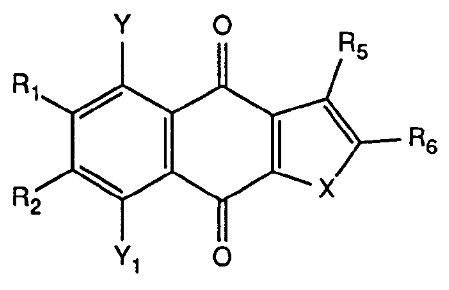

(III),

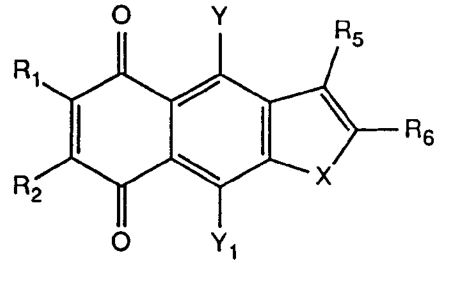

(IV),

worin $R_1$, $R_2$, $R_5$, $R_6$ und X die zuvor angegebenen Bedeutungen haben, Y für Halogen oder $-NO_2$ steht und $Y_1$ H, Halogen oder $-NO_2$ bedeutet, in Gegenwart eines polaren aprotischen oder protischen Lösungsmittels und bei erhöhter Temperatur mit mindestens äquivalenten Mengen einer Verbindung der Formel $R_9O^{\ominus}M^{\oplus}$ umsetzt, worin $R_9$ die zuvor angegebenen Bedeutungen hat und $M^{\oplus}$ für ein Alkalimetall- oder ein Ammoniumkation steht.

17. Verbindungen der Formeln m und IV

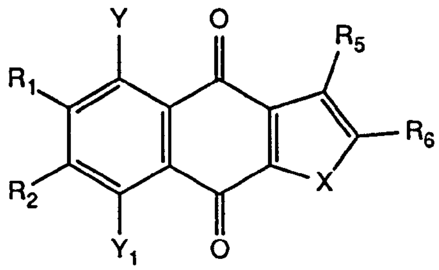

(III),

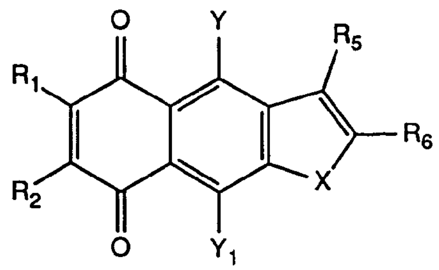

(IV),

worin $R_1$, $R_2$, $R_5$, $R_6$ und X die in Anspruch 1 angegebenen Bedeutungen haben, Y für Halogen oder $-NO_2$ steht und $Y_1$ H, Halogen oder $-NO_2$ bedeutet.

**18.** Verbindungen der Formeln V und VI

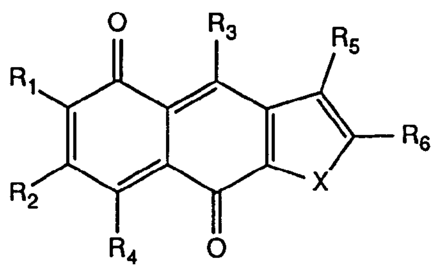

(V),

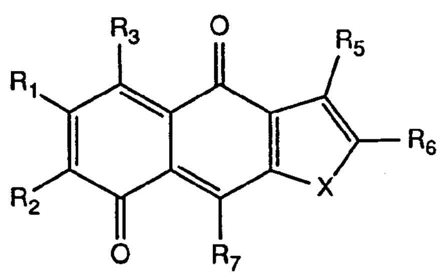

(VI),

worin X und $R_1$ bis $R_7$ die in Anspruch 1 angegebenen Bedeutungen haben.

**19.** Strahlungsempfindliche Zusammensetzung, enthaltend
a) ein strahlungsempfindliches organisches Material, und
b) mindestens eine Verbindung der Formeln I oder II, beziehungsweise V oder VI oder Mischungen davon.

**20.** Zusammensetzung gemäss Anspruch 19, dadurch gekennzeichnet, dass die Verbindungen der Formel I oder II und V oder VI oder Mischungen davon in einer Menge von 0,001 bis 20 Gew.-% enthalten sind, bezogen auf die Komponente a).

**21.** Zusammensetzung, enthaltend
a) ein farbloses organisches Lösungsmittel, ein Polymer oder ein organisches Glas oder ein Verbundglas, und
b) gelöst, eingemischt oder als Schicht auf mindestens einer Oberfläche eine Verbindung der Formel I oder II beziehungsweise V oder VI oder Mischungen davon.

**22.** Zusammensetzung gemäss Anspruch 21, dadurch gekennzeichnet, das die Komponente b) in einer Menge von 0,001 bis 20 Gew.-% enthalten ist, bezogen auf Komponente a).

**23.** Verfahren zur Herstellung von gefarbten Materialien unter Einwirkung von Licht, dadurch gekennzeichnet, dass man dem Material eine Verbindung der Formel I oder II beziehungsweise V oder VI einverleibt und darauf das Material mit Licht bestrahlt.

**24.** Verwendung von Verbindungen der Formeln I oder II beziehungsweise V oder VI als reversible photochrome Systeme zur Kontrastbildung oder Lichtabsorption.

**25.** Verwendung von Verbindungen der Formel I oder II beziehungsweise V oder VI als Farbindikatoren oder photoschaltbare Farbfilter bei Lichteinwirkung.

**26.** Verwendung der Verbindung der Formel I oder II beziehungsweise V oder VI zur reversiblen optischen Speicherung von Information, wobei die Information in einer die Verbindung enthaltenden, speicheraktiven Schicht mit Licht, bevorzugt Laserlicht, eingeschrieben wird.

Europäisches Patentamt

# EUROPÄISCHER RECHERCHENBERICHT

Nummer der Anmeldung

EP 93 81 0689
PAGE1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,D | JOURNAL OF ORGANIC CHEMISTRY<br>Bd. 24, Nr. 11, 1959, EASTON US<br>Seiten 1756 - 1759<br>M.F. SARTORI<br>* Beispiele X,IV * | 17 | C07D333/74<br>C07D209/60<br>C07D209/88<br>C07D307/92<br>C09K9/02<br>C09B5/26<br>C09B5/24<br>G03C1/73 |
| X | US-A-2 501 131 (H.R. LEE ET AL.)<br>* Anspruch 1 * | 17 | |
| X | US-A-2 513 574 (H.R. LEE ET AL.)<br>* Beispiele 8,9 * | 17 | |
| X | US-A-2 513 790 (L.F. FIESER ET AL.)<br>* Beispiel 1 * | 17 | |
| Y | DE-B-1 793 539 (RANK XEROX LTD.)<br>* Ansprüche * | 1 | |
| Y | US-A-3 033 879 (M.F. SARTORI)<br>* Spalte 2 * | 1 | **RECHERCHIERTE SACHGEBIETE (Int. Cl.5)** |
| Y | FR-A-2 383 940 (IMPERIAL CHEMICAL INDUSTRIES LTD.)<br>* Ansprüche * | 1 | C07D<br>C09K<br>C09B<br>G03C<br>C07C |
| Y | CHEMICAL ABSTRACTS, vol. 75, no. 1, 5. Juli 1971, Columbus, Ohio, US;<br>abstract no. 5051a,<br>* Zusammenfassung *<br>& ZH. VSES. KHIM. OBSHCHEST.<br>Bd. 16, Nr. 1, 1971,<br>Seite 105<br>YU.E. GERASIMENKO ET AL. | 1 | |
| | -/-- | | |

Der vorliegende Recherchenbericht wurde für alle Patentansprüche erstellt

| Recherchenort | Abschlußdatum der Recherche | Prüfer |
|---|---|---|
| BERLIN | 23 DEZEMBER 1993 | FRELON D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 93 81 0689
PAGE2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | CHEMICAL ABSTRACTS, vol. 96, no. 7, 15. Februar 1982, Columbus, Ohio, US; abstract no. 51665k, * Zusammenfassung * & IZV. SIB. OTD. AKAD. NAUK SSSR, SER. KHIM. NAUK Nr. 5, 1981, Seiten 116 - 125 E.P. FOKIN ET AL. --- | 1 | |
| A | CHEMICAL ABSTRACTS, vol. 100, no. 9, 27. Februar 1984, Columbus, Ohio, US; abstract no. 67607e, * Zusammenfassung * & TEOR. EKSP. KHIM. Bd. 19, Nr. 5, 1983, Seiten 577 - 584 N.P. GRITSAN ET AL. --- | 1 | |
| A | CHEMICAL ABSTRACTS, vol. 94, no. 7, 16. Februar 1981, Columbus, Ohio, US; abstract no. 47012m, * Zusammenfassung * & ZH. ORG. KHIM. Bd. 16, Nr. 9, 1980, Seiten 1938 - 1945 YU.E. GERASIMENKO ET AL. --- | 1 | RECHERCHIERTE SACHGEBIETE (Int. Cl.5) |
| A,D | EP-A-0 438 376 (CIBA-GEIGY A.G.) * Zusammenfassung * --- | 1 | |
| A | DE-A-2 855 939 (BAYER A.G.) * Seite 4 - Seite 5 * --- | 1 | |

-/--

Der vorliegende Recherchenbericht wurde für alle Patentansprüche erstellt

| Recherchenort | Abschlußdatum der Recherche | Prüfer |
|---|---|---|
| BERLIN | 23 DEZEMBER 1993 | FRELON D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung
EP 93 81 0689
PAGE3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | DATABASE WPI<br>Week 8123,<br>Derwent Publications Ltd., London, GB;<br>AN 79-13096B<br>& JP-B-56 019 862 (MITSUBISHI CHEM. IND. K.K.)<br>* Zusammenfassung *<br>--- | 16 | |
| A | CHEMICAL ABSTRACTS, vol. 104, no. 9, 3. März 1986, Columbus, Ohio, US;<br>abstract no. 68640e,<br>* Zusammenfassung *<br>& CS-A-225 285 (Z. KYPENA ET AL.)<br>--- | 16 | |
| A | DATABASE WPI<br>Week 8642,<br>Derwent Publications Ltd., London, GB;<br>AN 86-276588<br>& JP-A-61 203 194 (SUMITOMO CHEM. IND. K.K.)<br>* Zusammenfassung *<br>----- | 24 | RECHERCHIERTE SACHGEBIETE (Int. Cl.5 ) |

Der vorliegende Recherchenbericht wurde für alle Patentansprüche erstellt

| Recherchenort | Abschlußdatum der Recherche | Prüfer |
|---|---|---|
| BERLIN | 23 DEZEMBER 1993 | FRELON D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)